# EUROPÄISCHE PATENTSCHRIFT

(11) **EP 0 433 836 B1**
(45) Veröffentlichungstag und Bekanntmachung des Hinweises auf die Patenterteilung: **27.03.1996**
(21) Anmeldenummer: 90123724.8
(22) Anmeldetag: 10.12.1990
(51) Int. Cl.: C07D 319/06, C09K 19/34

(54) **Halophenyl-substituierte Dioxane**
Halophenyl substituted dioxanes
Dioxanes substitués par des halophényls

(30) Priorität: 19.12.1989 CH 4539/89
(43) Veröffentlichungstag der Anmeldung: 26.06.1991
(73) Patentinhaber: F. HOFFMANN-LA ROCHE AG, CH-4002 Basel (CH)
(72) Erfinder: Schadt, Martin, Dr., CH-4411 Seltisberg (CH); Villiger, Alois, Dr., CH-4055 Basel (CH)
(74) Vertreter: Cottong, Norbert A.

(56) Entgegenhaltungen:
- EP-A- 0 122 389
- EP-A- 0 315 014
- LIQUID CRYSTALS, Band 7, Nr. 4, 1990, Seiten 519-536; M. SCHADT et al.: "Synergisms, structure-material relations and display performance of novel fluorinated alkenyl liquid crystals"

## Beschreibung

Die vorliegende Erfindung betrifft neue halophenyl-substituierte Dioxane, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannung und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsbereich unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. So sind beispielsweise in EP-A 122 389 1E- und 3E-Alkenylderivate und in EP-A 315 014 Halophenylderivate offenbart, welche sich für obige Zwecke eignen. Seit einigen Jahren besteht ein besonderes Interesse an aktiv adressierten Flüssigkristallanzeigen, z.B. TFT-Anwendungen ("thin film transistor" = Dünnfilmtransistor) in Fernsehgeräten. Die Verwendung von Cyano-Verbindungen mit positiver dielektrischer Anisotropie führt jedoch in solchen Anzeigen meist zu einem unerwünscht hohen Stromanstieg.

Da Flüssigkristalle in der Regel als Mischungen mehrerer Komponenten zur Anwendung gelangen, ist es wichtig, dass die Komponenten untereinander gut mischbar sind.

Gegenstand der Erfindung sind die Verbindungen der allgemeinen Formel 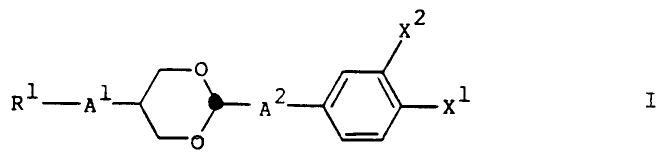
worin A¹ eine einfache Kovalenzbindung und A² 1,4-Phenylen oder trans-1,4-Cyclohexylen bezeichnen oder A¹ trans-1,4-Cyclohexylen und A² eine einfache Kovalenzbindung bezeichnen; X¹ Fluor oder Chlor bedeutet; X² Fluor oder, wenn X¹ Chlor bedeutet, auch Wasserstoff oder Chlor darstellt; und R¹ 1E-Alkenyl mit 2 bis 12 Kohlenstoffatomen bedeutet.

Die erfindungsgemässen Verbindungen sind Flüssigkristalle mit hohen Klärpunkten. Zudem werden hochgeordnete smektische Phasen werden ganz oder weitgehend unterdrückt. Sie weisen überraschend niedrige Viskositäten, insbesondere niedrige Rotationsviskositäten auf und sind gut mischbar mit üblichen Flüssigkristallmaterialien. Trotz der relativ schwachen permanenten Dipolmomente besitzen sie erstaunlich grosse positive dielektrische Anisotropien. Sie sind diesbezüglich zum Teil sogar vergleichbar mit bicyclischen Cyano-Verbindungen, welche aber niedrigere Klärpunkte und höhere Viskositäten aufweisen und bei TFT-Anwendungen zu Leitfähigkeitsproblemen führen. Die erfindungsgemässen Verbindungen ermöglichen daher tiefe Schwellenspannungen und trotzdem kurze Schaltzeiten.

Die erfindungsgemässen Verbindungen eignen sich insbesondere als Komponenten nematischer und cholesterischer Gemische und können dank ihrer guten Löslichkeit untereinander und in bekannten Flüssigkristallen auch in vergleichsweise hohen Konzentrationen verwendet werden.

Die Eigenschaften können je nach Bedeutung der Gruppen bis zu einem gewissen Grad variiert werden. Ein Cyclohexanring in A¹ oder A² führt zu vergleichsweise niedriger optischer Anisotropie. Ein Benzolring in A² führt zu vergleichsweise hoher optischer Anisotropie. Difluor-Derivate (X¹=X²=F) ergeben besonders grosse dielektrische Anisotropien und vergleichsweise kurze Schaltzeiten. p-Chlorderivate (X¹ = Cl) ergeben in der Regel höhere Klärpunkte, und ähnlich kurze Schaltzeiten.

Der Ausdruck "1E-Alkenyl" umfasst geradkettige und verzweigte, gegebenenfalls chirale Reste. Die geradkettigen Reste Vinyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undecenyl und 1E-Dodencenyl sind im allgemeinen bevorzugt.

Formel I umfasst die Verbindungen der allgemeinen Formeln 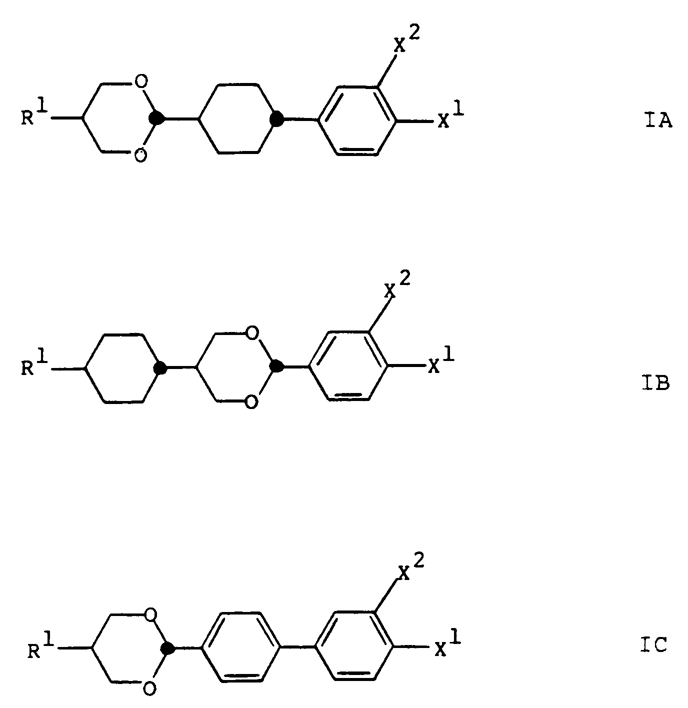
worin R¹, X¹ und X² die obigen Bedeutungen haben.

Von den Verbindungen der obigen Formeln I, IA, IB und IC sind im allgemeinen diejenigen bevorzugt, worin X¹ und X² Fluor bedeuten, wenn eine grosse dielektrische Anisotropie erwünscht ist, und diejenigen, worin X¹ Chlor und X² Wasserstoff bedeuten, wenn vergleichsweise hohe Klärpunkte erwünscht sind.

Bevorzugte Reste R¹ sind diejenigen mit 2 bis 7 Kohlenstoffatomen, insbesondere diejenigen mit 2 bis 5 Kohlenstoffatomen.

Die Verbindungen der Formel I können erfindungsgemäss dadurch hergestellt werden, dass man einen Aldehyd der allgemeinen Formel 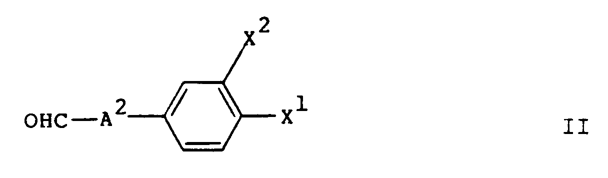
oder ein Acetal hiervon mit einer Verbindung der allgemeinen Formel 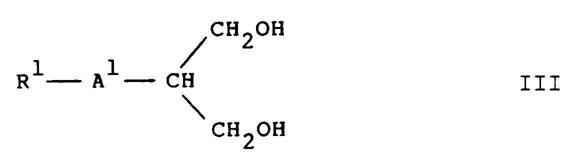
worin R¹, A¹, A², X¹ und X² die obigen Bedeutungen haben,
umsetzt.

Die Umsetzung des Aldehyds der Formel II oder eines geeigneten Acetals (z.B. Dimethylacetal oder Aethylenacetal) mit dem Diol der Formel III kann in an sich bekannter Weise erfolgen. Vorzugsweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure, Schwefelsäure oder trockenem Chlorwasserstoff. Temperatur und Druck snd nicht kritisch. Vorzugsweise wird jedoch bei Atmosphärendruck und Rückflusstemperatur unter Abtrennung des gebildeten Wassers gearbeitet.

Die Ausgangsmaterialien der Formeln II und III sind bekannte oder Analoge bekannter Verbindungen und können nach bekannten Methoden hergestellt werden. Geeignete Methoden sind in den Synthesebeispielen illustriert.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxan, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Die Verbindungen der Formel I eignen sich insbesondere für nematische oder, sofern mindestens eine Komponente des Gemisches optisch aktiv ist, auch für cholesterische Gemische.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formel 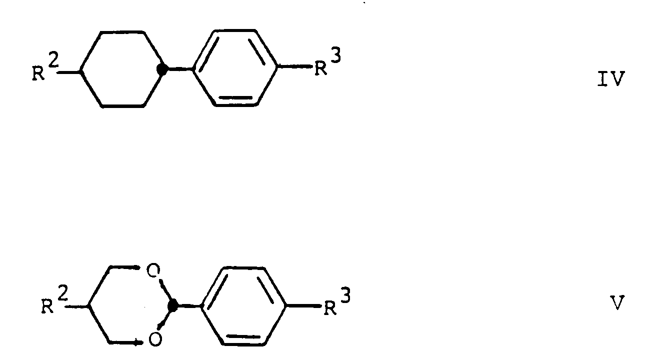 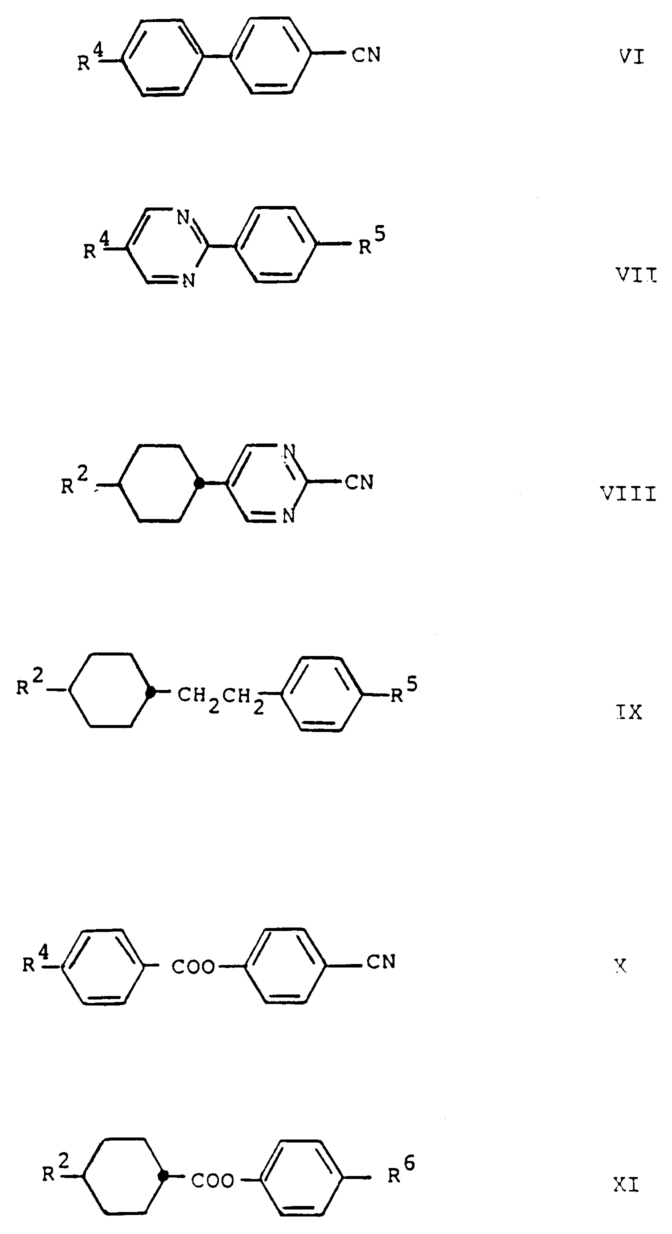 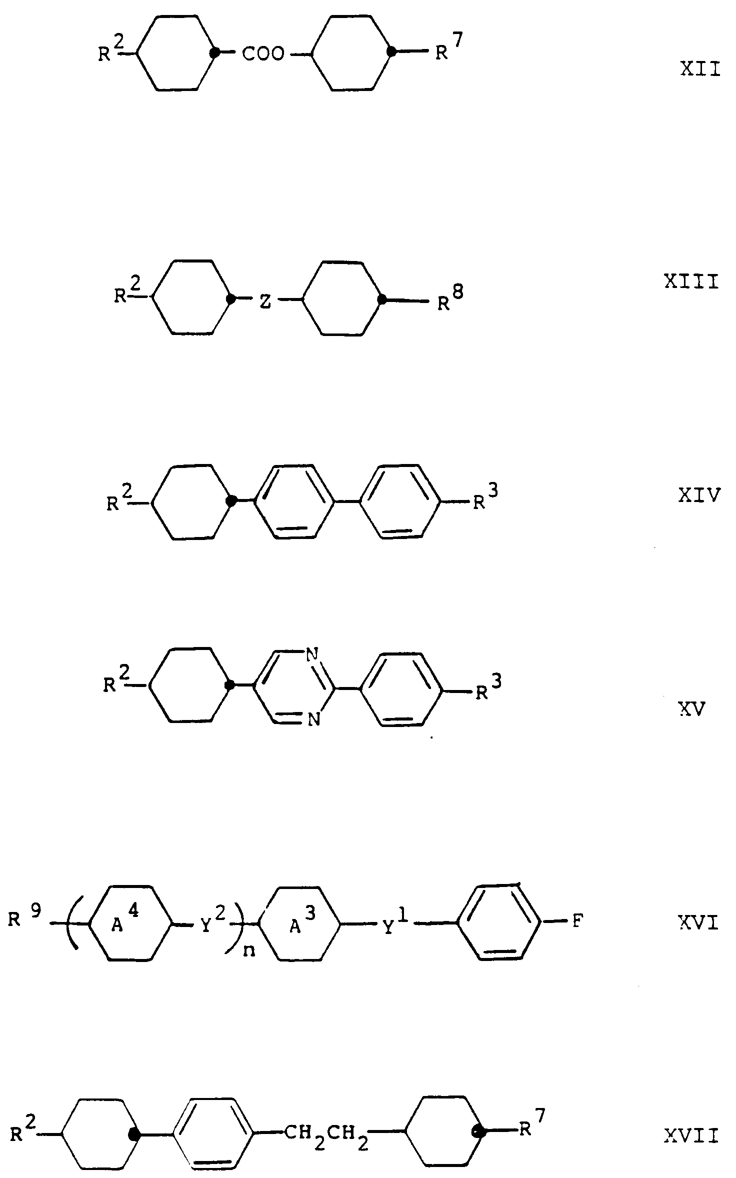 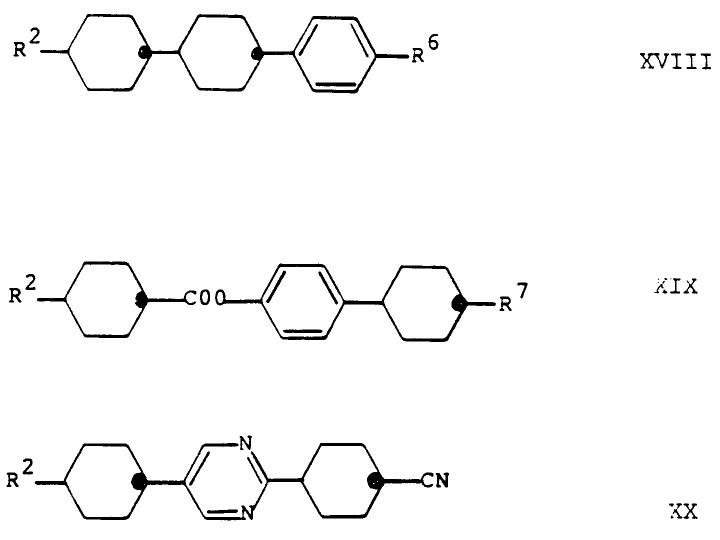
worin R² und R⁷ Alkyl, 1E-Alkenyl, 3E-Alkenyl oder 4-Alkenyl bedeuten; R³ Cyano, -NCS, Alkyl, 1E-Alkenyl, 3E-Alkenyl, 4-Alkenyl, Alkoxy, 2E-Alkenyloxy oder 3-Alkenyloxy bedeutet; R⁴ Alkyl, 3E-Alkenyl oder 4-Alkenyl bedeutet; R⁵ Cyano, -NCS, Alkyl, 3E-Alkenyl, 4-Alkenyl, Alkoxy, 2E-Alkenyloxy oder 3-Alkenyloxy bedeutet; R⁶ Cyano, Alkyl, 3E-Alkenyl, 4-Alkenyl, Alkoxy, 2E-Alkenyloxy oder 3-Alkenyloxy bedeutet; Z eine einfache Kovalenzbindung oder -CH₂CH₂- bedeutet; R⁸ Cyano, Alkoxy, 2E-Alkenyloxy, 3-Alkenyloxy, Alkyl, 1E-Alkenyl, 3E-Alkenyl, 4-Alkenyl, Alkoxymethyl oder 2-Alkenyloxymethyl bedeutet; n für die Zahl 0 oder 1 steht; eine der Gruppen Y¹ und Y² eine einfache Kovalenzbindung, -COO-, -OOC-, -CH₂CH₂-, -CH₂O- oder -OCH₂-und die andere der Gruppen Y¹ und Y² eine einfache Kovalenzbindung bedeutet; die Ringe A³ und A⁴ unabhängig voneinander substituiertes oder unsubstituiertes trans-1,4-Cyclohexylen, in welchem gegebenenfalls 2 nicht benachbarte CH₂-Gruppen durch Sauerstoff ersetzt sind, oder substituiertes oder unsubstituiertes 1,4-Phenylen, in welchem gegebenenfalls 1 oder 2 CH-Gruppen durch Stickstoff ersetzt sind, darstellen; R⁹ Alkyl, 1E-Alkenyl oder, wenn eine der Gruppen Y¹ und Y² eine einfache Kovalenzbindung, -COO-, -OOC- oder -CH₂CH₂- und die andere der Gruppen Y¹ und Y² eine einfache Kovalenzbindung bedeutet, auch 3E-Alkenyl bezeichnet.

Die Alkyl-, Alkenyl-, Alkoxy- und Alkenyloxyreste R²-R⁹ in den Formeln IV-XX sind vorzugsweise geradkettige Reste. Sie weisen vorzugsweise bis zu 12, besonders bevorzugt bis zu 7 Kohlenstoffatome auf.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Bespiele weiter veranschaulicht. C bedeutet eine kristalline, S_{A} eine smektisch A, S_{B} eine smektisch B. N eine nematische und I eine isotrope Phase. V₁₀ und V₅₀ bezeichnen die Spannung für 10% bzw. 50% Transmission, tₒₙ und t_{off} bezeichnen die Einschaltzeit bzw. die Ausschaltzeit und △n bezeichnet die optische Anisotropie. k₁₁ und k₃₃ bedeuten die elastischen Konstanten für Spreizung bzw. Biegung. △ε bezeichnet die dielektrische Anisotriopie, η die Bulk-Viskosität und γ₁ die Rotationsviskosität. Die elektro-optischen Eigenschaften wurden bei 22°C oder bei einer Temperatur 10°C unterhalb des Klärpunktes gemessen. Sofern nichts angegeben ist, erfolgte die Messung bei 22°C.

### Beispiel 1

Eine Lösung von 3,0 g trans-4-(3,4-Difluorphenyl)-cyclohexancarboxaldehyd (hergestellt gemäss Beispiel 2) und 2,4 g 2-(1E-Propenyl)-1,3-propandiol in 75 ml Toluol wurde mit 3 Tropfen 10-prozentiger (v/v) Schwefelsäure versetzt und das Reaktionsgemisch 1,25 Stunden zum Sieden erhitzt, wobei feuchtes Toluol abdestilliert und durch frisches Toluol ersetzt wurde. Dann wurde das Reaktionsgemisch mit Triäthylamin neutralisiert und nach dem Erkalten dreimal mit Wasser gewaschen, über Natriumsulfat getrocknet und eingeengt. Chromatographische Reinigung des Rückstandes an 100 g Kieselgel mit Hexan/Aethylacetat (Vol. 49:1) und mehrmalige Umkristallisation des trans/cis-Gemisches aus Hexan ergab 0,8 g reines trans-5-(1E-Propenyl)-2-[trans-4-(3,4-difluorphenyl)cyclohexyl]-1,3-dioxan; Smp. (C-N) 96,0°C, Klp. (N-I) 125,5°C, △ε (115°C) = 12,4.

In analoger Weise können folgende Verbindungen hergestellt werden:
trans-5-Vinyl-2-[trans-4-(3,4-difluorphenyl)cyclohexyl]-1,3-dioxan;
trans-5-(1E-Butenyl)-2-[trans-4-(3,4-difluorphenyl)cyclohexyl]-1,3-dioxan, Smp. (C-S_{B}) 42,7°C, S_{B}-N 79,5°C, Klp. (N-I) 117,6°C;
trans-5-(1E-Pentenyl)-2-[trans-4-(3,4-difluorphenyl)cyclohexyl]-1,3-dioxan; Smp. (C-S_{B}) 68.8°C, S_{B}-N 81.0°C, Klp. (N-I) 116°C;
trans-5-(1E-Heptenyl)-2-[trans-4-(3,4-difluorphenyl)cyclohexyl]-1,3-dioxan;
trans-5-Vinyl-2-[trans-4-(4-chlorphenyl)cyclohexyl]-1,3-dioxan;
trans-5-(1E-Propenyl)-2-[trans-4-(4-chlorphenyl)cyclohexyl]-1,3-dioxan, Smp. (C-N) 101°C, Klp. (N-I) 183°C;
trans-5-(1E-Butenyl)-2-[trans-4-(4-chlorphenyl)cyclohexyl]-1,3-dioxan;
trans-5-(1E-Pentenyl)-2-[trans-4-(4-chlorphenyl)cyclohexyl]-1,3-dioxan, Smp. (C-S_{B}) 102,4°C, S_{B}-N 117°C, Klp. (N-I) 172,5°C;
trans-5-(1E-Propenyl)-2-[trans-4-(4-chlor-3-fluorphenyl)-cyclohexyl]-1,3-dioxan, Smp. (C-N) 81.9°C, Klp. (N-I) 141.5°C;
trans-5-(1E-Pentenyl)-2-[trans-4-(4-chlor-3-fluorphenyl)-cyclohexyl]-1,3-dioxan, Smp. (C-N) 90.4°C, S_{B}-N 78,7°C, Klp. (N-I) 139.5°C;
trans-5-(1E-Propenyl)-2-[trans-4-(3,4-dichlorphenyl)-cyclohexyl]-1,3-dioxan;
trans-5-Vinyl-2-(3',4'-difluor-4-biphenylyl)-1,3-dioxan;
trans-5-(1E-Propenyl)-2-(3',4'-difluor-4-biphenylyl)-1,3-dioxan, Smp. (C-N) 106,9°C, S_{A}-N 106.5°C, Klp. (N-I) 148,5°C;
trans-5-(1E-Butenyl)-2-(3',4'-difluor-4-biphenylyl)-1,3-dioxan, Smp. (C-S_{A}) 104.6°C, S_{A}-N 131.5°C, Klp. (N-I) 145°C;
trans-5-(1E-Pentenyl)-2-(3',4'-difluor-4-biphenylyl)-1,3-dioxan, Smp. (C-S_{A}) 79.6°C, S_{A}-N 137.5°C, Klp. (N-I) 148.5°C;
trans-5-(1E-Heptenyl)-2-(3',4'-difluor-4-biphenylyl)-1,3-dioxan;
trans-5-Vinyl-2-(4'-chlor-4-biphenylyl)-1,3-dioxan;
trans-5-(1E-Propenyl)-2-(4'-chlor-4-biphenylyl)-1,3-dioxan;
trans-5-(1E-Butenyl)-2-(4'-chlor-4-biphenylyl)-1,3-dioxan;
trans-5-(1E-Butenyl)-2-[trans-4-(4-chlor-3-fluorphenyl)-cyclohexyl]-1,3-dioxan, Smp. (C-N) 70.7°C, S_{B}-N 62°C, Klp (N-I) 134.5°C;
trans-5-(1E-Pentenyl)-2-(4'-chlor-4-biphenylyl)-1,3-dioxan;
trans-5-(1E-Propenyl)-2-(4'-chlor-3'-fluor-4-biphenylyl)-1,3-dioxan;
trans-5-(1E-Pentenyl)-2-(4'-chlor-3'-fluor-4-biphenylyl)-1,3-dioxan;
trans-5-(1E-Propenyl)-2-(3',4'-dichlor-4-biphenylyl)-1,3-dioxan;
trans-5-[trans-4-Vinylcyclohexyl]-2-(3,4-difluorphenyl)-1,3-dioxan;
trans-5-[trans-4-(1E-Propenyl)cyclohexyl]-2-(3,4-difluorphenyl)-1,3-dioxan;
trans-5-[trans-4-(1E-Pentenyl)cyclohexyl]-2-(3,4-difluorphenyl)-1,3-dioxan;
trans-5-[trans-4-Vinylcyclohexyl]-2-(4-chlorphenyl)-1,3-dioxan;
trans-5-[trans-4-(1E-Propenyl)cyclohexyl]-2-(4-chlorphenyl)-1,3-dioxan;
trans-5-[trans-4-(1E-Pentenyl)cyclohexyl]-2-(4-chlorphenyl)-1,3-dioxan;
trans-5-[trans-4-Vinylcyclohexyl]-2-(4-chlor-3-fluorphenyl)-1,3-dioxan;
trans-5-[trans-4-(1E-Propenyl)cyclohexyl]-2-(4-chlor-3-fluorphenyl)-1,3-dioxan;
trans-5-[trans-4-(1E-Propenyl)cyclohexyl]-2-(3,4-dichlorphenyl)-1,3-dioxan.

### Beispiel 2

a) Zu einer aus 1,21 g Magnesium, 9,45 g 1-Brom-3,4-difluorbenzol und 35 ml Tetrahydrofuran bereiteten Grignard-Reagens-Lösung wurde bei 5°C innert 30 Minuten eine Lösung von 7,47 g 1,4-Dioxa-8-spiro[4.5]decanon in 40 ml Tetrahydrofuran getropft. Das Reaktionsgemisch wurde noch 3 Stunden bei Raumtemperatur und 2 Stunden bei Siedehitze gerührt. Die rotbraune Lösung wurde mit 80 ml Diäthyläther verdünnt, einmal mit 60 ml 1N Ammoniumchlorid-Lösung und dreimal je 40 ml Wasser gewaschen, über Natriumsulfat getrocknet und eingeengt. Es resultierten 11,20 g festes 8-(3,4-Difluorphenyl)-1,4-dioxa-8-spiro-[4.5]decanol.
b) Ein Gemisch von 11,2 g 8-(3,4-Difluorphenyl)-1,4-dioxa-8-spiro[4.5]decanol, 300 ml Toluol, 2,4 ml Aethylenglykol und 2,1 g Amberlyst® 15 (stark saurer Ionenaustauscher, Fluka AG) wurde 3,5 Stunden am Wasserabscheider gekocht. Dann wurde das Reaktionsgemisch mit 1 ml Triäthylamin neutralisiert, 5 Minuten gerührt und vom Ionenaustauscher abfiltriert. Das Filtrat wurde zweimal mit je 40 ml Wasser gewaschen, über Natriumsulfat getrocknet und eingeengt. Chromatographische Reinigung des Rückstandes an 70 g Kieselgel mit Hexan/Aethylacetat (Vol. 9:1) ergab 6,44 g festes 8-(3,4-Difluorphenyl)-1,4-dioxa-7-spiro[4.5]decen.
c) Eine Lösung aus 6,44 g 8-(3,4-Difluorphenyl)-1,4-dioxa-7-spiro[4.5]decen, 1 ml Triäthylamin und 125 ml Toluol/Aceton (Vol. 4:1) wurde mit 1 g Palladium/Kohle (10%) bei Raumtemperatur und Normaldruck bis zum Stillstand der Wasserstoffaufnahme hydriert. Das Reaktionsgemisch wurde vom Katalysator abfiltriert und das Filtrat eingeengt. Es resultieren 6,30 g farbloses, festes 8-(3,4-Difluorphenyl)-1,4-dioxaspiro[4.5]decan.
d) Ein Gemisch aus 6,30 g 8-(3,4-Difluorphenyl)-1,4-dioxaspiro[4.5]decan, 60 ml Toluol und 40 ml Ameisensäure wurde 18 Stunden gerührt. Die Ameisensäure-Phase wurde abgetrennt und zweimal mit je 30 ml Toluol extrahiert. Die vereinigte Toluolphase wurde mit Wasser neutral gewaschen, über Natriumsulfat getrocknet und eingeengt. Es resultierten 5,00 g 4-(3,4-Difluorphenyl)cyclohexanon als farbloses Oel.
e) In Inertgasatmosphäre wurden 4,1 g Kalium-t-butylat zu einer Suspension von 12,2 g trockenem Methoxymethyltriphenylphosphoniumchlorid in 125 ml t-Butylmethyläther gegeben und die orangerote Suspension 30 Minuten gerührt. Bei 0-5°C wurde innert 1 Stunde eine Lösung von 5,00 g 4-(3,4-Difluorphenyl)cyclohexanon in 75 ml t-Butylmethyläther zugetropft. Das Reaktionsgemisch wurde noch 1 Stunde bei 0-5°C und 2,5 Stunden bei Raumtemperatur gerührt und dann mit 50 ml gesättigter Natriumhydrogencarbonat-Lösung versetzt. Die organische Phase wurde mit Wasser gewaschen und eingeengt. Der Rückstand wurde in 200 ml Hexan und 100 ml 80-prozentigem, wässrigem Methanol aufgenommen. Die Hexanphase wurde noch zweimal mit je 40 ml 80-prozentigem Methanol gewaschen, über Natriumsulfat getrocknet und eingeengt. Es resultierten 5,6 g 1,2-Difluor-4-[4-(methoxymethyliden)cyclohexyl]benzol.
f) Eine Lösung von 5,6 g 1,2-Difluor-4-[4-(methoxymethyliden)cyclohexyl]benzol in 125 ml Tetrahydrofuran/2N Salzsäure (Vol. 4:1) wurde 1,25 Stunden auf 90°C erhitzt. Das Reaktionsgemisch wurde anschliessend auf Eiswasser gegossen und mit Diäthyläther extrahiert. Die Aetherphase wurde mit 50 ml halbgesättigter Natriumhydrogencarbonat-Lösung und zweimal je 50 ml Wasser neutral gewaschen, über Natriumsulfat getrocknet und eingeengt. Eine Lösung des erhaltenen rohen trans/cis-Gemisches (5,1 g) in 25 ml Methanol wurde in Inertgasatmosphäre bei 0-3°C innert 5 Minuten zu 70 ml 0,1 N methanolischer Kaliumhydroxid-Lösung zugetropft. Das Reaktionsgemisch wurde 1 Stunde bei 0°C gerührt, dann auf 200 ml Eiswasser gegossen und mit Diäthyläther extrahiert. Die Aetherphasen wurden mit Wasser neutral gewaschen, über Natriumsulfat getrocknet und eingeengt. Es resultierten 4,8 g trans-4-(3,4-Difluorphenyl)cyclohexancarboxaldehyd (cis-Anteil 5,7%).

In analoger Weise können folgende Verbindungen hergestellt werden:
trans-4-(4-Chlorphenyl)cyclohexancarboxaldehyd,
trans-4-(4-Chlor-3-fluorphenyl)cyclohexancarboxaldehyd,
trans-4-(3,4-Difluorphenyl)cyclohexancarboxaldehyd.

### Beispiel 3

a) Zu einem Gemisch aus 9,0 g 2-(p-Iodphenyl)dioxolan, 100 ml Tetrahydrofuran, 0,025 g Palladium(II)chlorid und 0,16 g 1,3-Bis(triphenylphosphino)propan tropfte man eine aus 1,0 Magnesium, 7,6 g 1-Brom-3,4-difluorbenzol und 75 ml Tetrahydrofuran bereitete Grignard-Reagens-Lösung. Das Reaktionsgemisch wurde 3 Tage zum Sieden erhitzt und nach dem Erkalten mit 200 ml Diäthyläther und 120 ml 0,5 N Ammoniumchlorid-Lösung ausgeschüttelt. Die organische Phase dreimal mit je 80 ml Wasser gewaschen, über Natriumsulfat getrocknet und eingeengt. Chromatographische Reinigung des Rückstandes an Kieselgel mit Hexan/Aethylacetat (Vol. 9:1) ergab reines 2-(3',4'-Difluor-4-biphenylyl)-dioxolan; Smp. 89-91°C.
b) Ein Gemisch aus 5,4 g 2-(3',4'-Difluor-4-biphenylyl)dioxolan, 100 ml Toluol und 20 ml Ameisensäure wurde 16 Stunden gerührt. Die Ameisensäure-Phase wurde abgetrennt und die Toluol-Phase zweimal mit je 50 ml gesättigter Natriumhydrogencarbonat-Lösung und dreimal mit je 75 ml Wasser neutral gewaschen, über Natriumsulfat getrocknet und eingeengt. Es resultieren 4,3 g p-(3,4-Difluorphenyl)-benzaldehyd.

In analoger Weise können folgende Verbindungen hergestellt werden:
p-(4-Chlorphenyl)benzaldehyd,
p-(4-Chlor-3-fluorphenyl)benzaldehyd,
p-(3,4-Difluorphenyl)benzaldehyd.

### Beispiel 4

a) Ein Gemisch aus 33,3 g 4-(1E-Pentenyl)cyclohexanon (hergestellt gemäss EP-A-168683), 42 ml Cyanoessigsäureäthylester, 300 ml Toluol und 2,9 g Piperidiniumacetat wird 1,5 Stunden am Wasserabscheider gekocht. Nach dem Erkalten wird das Reaktionsgemisch dreimal mit je 50 ml Wasser gewaschen, über Natriumsulfat getrocknet und eingeengt. Chromatographische Reinigung des Rückstandes an 750 g Kieselgel mit Hexan/Aethylacetat (Vol. 19:1) ergibt Cyano-[4-(1E-pentenyl)cyclohexyliden]essigsäureäthylester.
b) Zu einem Gemisch aus 6,7 g Natriumborhydrid und 120 ml Aethanol wird innert 40 Minuten bei 5-10°C eine Lösung von 46,0 g Cyano-[4-(1E-pentenyl)cyclohexyliden]essigsäureäthylester in 120 ml Aethanol getropft. Das Reaktionsgemisch wird 45 Minuten bei 5°C gerührt, dann mit 750 ml Wasser verdünnt und dreimal mit je 250 ml Diäthyläther extrahiert. Der Extrakt wird mit Wasser neutral gewaschen, über Natriumsulfat getrocknet und eingeengt. Es resultiert roher Cyano-[trans-4-(1E-pentenyl)cyclohexyl]essigsäureäthylester (cis-Anteil < 20%).
c) Ein Gemisch aus 36,9 g Kaliumhydroxid, 250 ml Wasser, 250 ml Aethanol und 37,2 g Cyano-[trans-4-(1E-pentenyl)cyclohexyl]essigsäureäthylester wird unter Rühren 3 Tage zum Sieden erhitzt. Das Reaktionsgemisch wird nach dem Erkalten mit 500 ml Wasser verdünnt und zweimal mit je 200 ml Diäthyläther gewaschen. Zur wässrigen Phase werden 220 ml 10-prozentige (v/v) Schwefelsäure zugetropft. Das ausgefallene Produkt wird dreimal mit je 200 ml Diäthyläther extrahiert. Der Extrakt wird zweimal mit je 50 ml Wasser gewaschen, über Natriumsulfat getrocknet, filtriert und eingeengt. Mehrmalige Umkristallisation aus Benzol/Hexan (Vol. 1:1) ergibt reine [trans-4-(1E-Pentenyl)cyclohexyl]malonsäure.
d) In Inertgasatmosphäre wird eine Lösung von 28,6 g [trans-4-(1E-Pentenyl)cyclohexyl]malonsäure in 150 ml absolutem Diäthyläther innert 1 Std. so zu einer Suspension von 8,6 g Lithiumaluminiumhydrid in 250 ml absolutem Diäthyläther zugetropft, dass das Gemisch nicht zu stark siedet. Man erhitzt noch 4 Stunden zum Sieden und tropft dann vorsichtig 15 ml eiskaltes Wasser und danach 250 ml 25-prozentige (v/v) Schwefelsäure zu, bis sich die wässrige Phase als grauer Schlamm absetzt. Die Aetherphase wird dekantiert und der Rückstand viermal mit je 200 ml Diäthyläther extrahiert. Die vereinigte Aetherphase wird einmal mit 25 ml 25-prozentiger Schwefelsäure, zweimal mit je 25 ml gesättigter Natriumhydrogencarbonat-Lösung und zweimal mit je 25 ml gesättigter Kochsalz-Lösung gewaschen, dann über Natriumsulfat getrocknet und eingeengt. Fraktionelle Destillation des Rückstandes im Hochvakuum ergibt reines 2-[trans-4-(1E-Pentenyl)cyclohexyl]-1,3-propandiol.

In analoger Weise können folgende Verbindungen hergestellt werden:
2-[trans-4-Vinylcyclohexyl]-1,3-propandiol,
2-[trans-4-(1E-Propenyl)cyclohexyl]-1,3-propandiol,
2-[trans-4-(1E-Butenyl)cyclohexyl]-1,3-propandiol.

### Beispiel 5

Zur Untersuchung der Eigenschaften der Verbindungen der Formel I in Gemischen wurden binäre Mischungen hergestellt. Die Schwellenspannung und die Ansprechzeiten wurden in einer TN-Zelle (low bias tilt) mit 8 µm Plattenabstand gemessen; als Betriebsspannung wurde der 2,5-fache Wert der Schwellenspannung gewählt.

### Mischung A

- 80 Mol-%: 4-(2E-Butenyloxy)-1-(trans-4-propylcyclohexyl)-benzol,
- 20 Mol-%: trans-5-(1E-Propenyl)-2-[trans-4-(3,4-difluorphenyl)cyclohexyl]-1,3-dioxan;
Smp. (C-N) 22°C, Klp. (N-I) 60,9°C, k₃₃/k₁₁ (50,9°C) = 1,21, △ε (22°C) = 3,314, △ε (50,9°C) = 2,378, △n = (50,9° C) = 0,085; η (22°C) = 15,8 cP; η (50,9°C) = 6,1 cP, γ₁ (50,9°C) = 24,5 cP; V₁₀ (22°C) = 2,6 V, V₁₀ (50,9°C) = 2,0 V; tₒₙ (22°C) = 11 ms, tₒₙ (50,9°C) = 6,2 ms, t_{off} (22°C) = 20 ms, t_{off} (50,9°C) = 11 ms.
Eigenschaften von reinem 4-(2E-Butenyloxy)-1-(trans-4-propylcyclohexyl)benzol: Smp. (C-N) 42,4°C, Klp. (N-I) 57,5°C; k₃₃/k₁₁ (47,5°C) = 1,16, △ε (47,5°C) = -0,268, △n (47,5°C) = 0,089; η (22°C) = 13,5 cP, η (47,5°C) = 4,7 cP, γ₁ (22°C) = 86 cP, γ₁ (47,5°C) = 25 cP.

### Mischung B

- 90 Gew.-%: 4-(trans-4-Pentylcyclohexyl)benzonitril,
- 10 Gew.-%: trans-5-(1E-Butenyl)-2-[trans-4-(3,4-difluorphenyl)cyclohexyl]-1,3-dioxan;
Klp. (N-I) 54,5°C, V₁₀ = 1,32 V, tₒₙ = 28 ms, t_{off} = 46 ms, △n = 0,120.

### Mischung C

- 80 Gew.-%: 4-(trans-4-Pentylcyclohexyl)benzonitril,
- 20 Gew.-%: trans-5-(1E-Butenyl)-2-[trans-4-(3,4-difluorphenyl)cyclohexyl]-1,3-dioxan;
Klp. (N-I) 54,6°C, V₁₀ = 1,24 V, tₒₙ = 33 ms, t_{off} = 53 ms, △n = 0,112.

### Mischung D

- 90 Gew.-%: 4-(trans-4-Pentylcyclohexyl)benzonitril,
- 10 Gew.-%: trans-5-(1E-Propenyl)-2-[trans-4-(3,4-difluorphenyl)cyclohexyl]-1,3-dioxan;
Klp. (N-I) 54,9°C, V₁₀ = 1,39 V, tₒₙ = 27 ms, t_{off} = 47 ms, △n = 0,118.
Eigenschaften von reinem 4-(trans-4-Pentylcyclohexyl)-benzontril: Klp. (N-I) 54,6°C, V₁₀ = 1,62 V, tₒₙ = 30 ms, t_{off} = 42 ms, △n = 0,120.

## Patentansprüche

1. Verbindungen der allgemeinen Formel 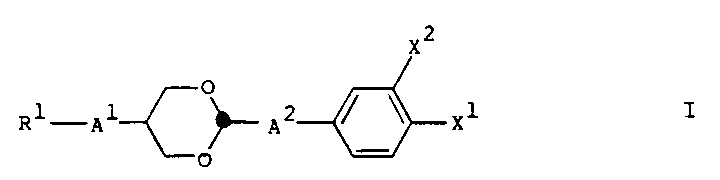 worin A¹ eine einfache Kovalenzbindung und A² 1,4-Phenylen oder trans-1,4-Cyclohexylen bezeichnen oder A¹ trans-1,4-Cyclohexylen und A² eine einfache Kovalenzbindung bezeichnen; X¹ Fluor oder Chlor bedeutet; X² Fluor oder, wenn X¹ Chlor bedeutet, auch Wasserstoff oder Chlor darstellt; und R¹ 1E-Alkenyl mit 2 bis 12 Kohlenstoffatomen bedeutet.

2. Verbindungen nach Anspruch 1, dadurch gekennzeichnet, dass R¹ ein geradkettiger Rest ist.

3. Verbindungen nach Anspruch 1 oder 2, dadurch gekennzeichnet, dass R¹ 1E-Alkenyl mit 2 bis 7 Kohlenstoffatomen vorzugsweise 2 bis 5 Kohlenstoffatomen bedeutet.

4. Verbindungen nach einem der Ansprüche 1 bis 3, dadurch gekennzeichnet, dass X¹ und X² Fluor bedeuten.

5. Verbindungen nach einem der Ansprüche 1 bis 3, dadurch gekennzeichnet, dass X¹ Chlor und X² Wasserstoff bedeuten.

6. Flüssigkristallines Gemisch mit mindestens zwei Komponenten, dadurch gekennzeichnet, dass mindestens eine Komponente eine Verbindung der in Anspruch 1 definierten Formel I ist.

7. Flüssigkristallines Gemisch nach Anspruch 6, dadurch gekennzeichnet, dass der Anteil an Verbindungen der Formel I 1-50, vorzugsweise 5-30 Gew.-% beträgt.

8. Verfahren zur Herstellung der Verbindungen der in Anspruch 1 definierten Formel I, dadurch gekennzeichnet, dass man einen Aldehyd der allgemeinen Formel 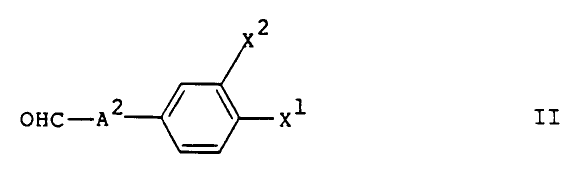 oder ein Acetal hiervon mit einer Verbindung der allgemeinen Formel 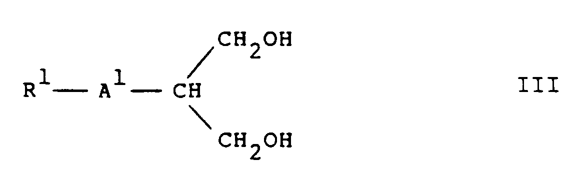 worin R¹, A¹, A², X¹, und X² die in Anspruch 1 gegebenen Bedeutungen haben,
umsetzt.

9. Verwendung der Verbindungen der in Anspruch 1 definierten Formel I für elektro-optische Zwecke.

## Claims

1. Compounds of the general formula 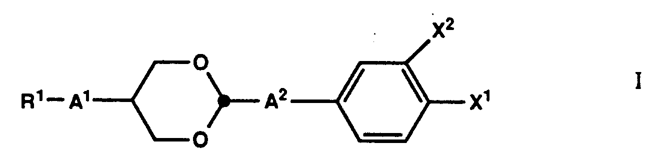 wherein A¹ denotes a single covalent bond and A² denotes 1,4-phenylene or trans-1,4-cyclohexylene or A¹ denotes trans-1,4-cyclohexylene and A² denotes a single covalent bond; X¹ signifies fluorine or chlorine; X² represents fluorine or, when X¹ signifies chlorine, also hydrogen or chlorine; and R¹ signifies 1E-alkenyl with 2 to 12 carbon atoms.

2. Compounds according to claim 1, characterized in that R¹ is a straight-chain residue.

3. Compounds according to claim 1 or 2, characterized in that R¹ signifies 1E-alkenyl with 2 to 7 carbon atoms, preferably 2 to 5 carbon atoms.

4. Compounds according to any one of claims 1 to 3, characterized in that X¹ and X² signify fluorine.

5. Compounds according to any one of claims 1 to 3, characterized in that X¹ signifies chlorine and X² signifies hydrogen.

6. A liquid crystalline mixture with at least two components, characterized in that at least one component is a compound of formula I defined in claim 1.

7. A liquid crystalline mixture according to claim 6, characterized in that the content of compounds of formula I is 1-50, preferably 5-30, wt.%.

8. A process for the manufacture of the compounds of formula I defined in claim 1, characterized by reacting an aldehyde of the general formula 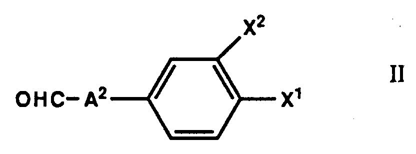 or an acetal thereof with a compound of the general formula 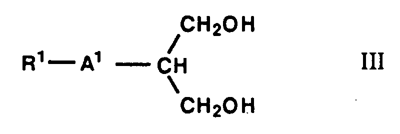 wherein R¹, A¹, A², X¹ and X² have the significances given in claim 1.

9. The use of the compounds of formula I defined in claim 1 for electro-optical purposes.

## Revendications

1. Composés de formule générale 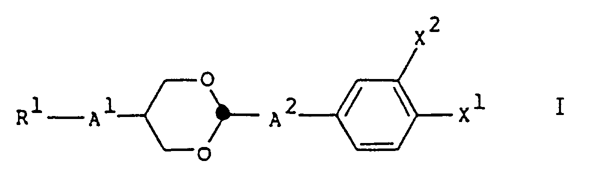 où A¹ représente une liaison covalente simple et A² le 1,4-phénylène ou le trans-1,4-cyclohexylène ou A¹ représente le trans-1,4-cyclohexylène et A² une liaison covalente simple ; X¹ représente le fluor ou le chlore ; X² représente le fluor ou, lorsque X¹ représente le chlore, X² représente également l'hydrogène ou le chlore ; et R¹ représente un 1E-alcényle comportant 2 à 12 atomes de carbone.

2. Composés selon la revendication 1, caractérisés en ce que R¹ est un reste linéaire.

3. Composés selon la revendication 1 ou 2, caractérisés en ce que R¹ représente un 1E-alcényle comportant 2 à 7 atomes de carbone, de préférence, 2 à 5 atomes de carbone.

4. Composés selon l'une des revendications 1 à 3, caractérisés en ce que X¹ et X² représentent le fluor.

5. Composés selon l'une des revendications 1 à 3, caractérisés en ce que X¹ représente le chlore et X² l'hydrogène.

6. Mélange à cristaux liquides comportant au moins deux composants, caractérisé en ce qu'au moins un composant est un composé de formule I définie dans la revendication 1.

7. Mélange à cristaux liquides selon la revendication 6, caractérisé en ce que la proportion en composés de formule I est comprise entre 1 et 50% en poids, de préférence entre 5 et 30% en poids.

8. Procédé pour la préparation des composés de formule I définie dans la revendication 1, caractérisé en ce que l'on fait réagir un aldéhyde de formule générale 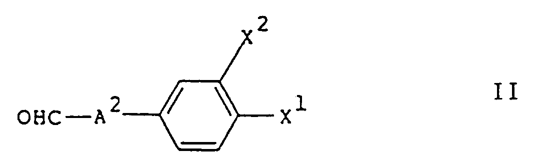 ou l'un de ses acétals avec un composé de formule générale 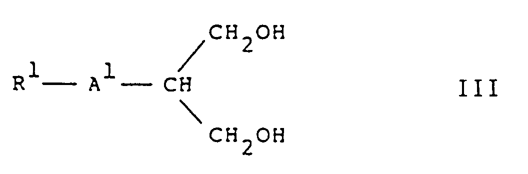 où R¹, A¹, A², X¹ et X² ont les significations données dans la revendication 1.

9. Utilisation des composés de formule I définie dans la revendication 1 à des fins électro-optiques.
